# EUROPÄISCHE PATENTSCHRIFT

(11) **EP 0 805 983 B1**
(45) Veröffentlichungstag und Bekanntmachung des Hinweises auf die Patenterteilung: **12.01.2000**
(21) Anmeldenummer: 96902934.7
(22) Anmeldetag: 29.01.1996
(51) Int. Cl.: G01N 33/543, G01N 33/532, G01R 33/12, A61K 49/00

(54) **VERFAHREN UND VERBINDUNGEN ZUR MAGNETORELAXOMETRISCHEN DETEKTION VON ANALYTEN UND DEREN VERWENDUNG**
PROCESS AND COMPOUNDS FOR THE MAGNETORELAXOMETRIC DETECTION OF ANALYTES AND USE THEREOF
PROCEDE ET COMPOSES PERMETTANT D'EFFECTUER UNE DETECTION MAGNETORELAXOMETRIQUE D'ANALYTES ET LEUR UTILISATION

(30) Priorität: 27.01.1995 DE 19503664
(43) Veröffentlichungstag der Anmeldung: 12.11.1997
(73) Patentinhaber: SCHERING AKTIENGESELLSCHAFT, 13353 Berlin (DE)
(72) Erfinder: WEITSCHIES, Werner, D-10961 Berlin (DE); KÖTITZ, Roman, D-13189 Berlin (DE); TRAHMS, Lutz, D-12103 Berlin (DE); BUNTE, Thomas, D-12307 Berlin (DE)
(86) Internationale Anmeldenummer: EP9600339
(87) Internationale Veröffentlichungsnummer: WO9623227

(56) Entgegenhaltungen:
- WO-A-91/15243
- WO-A-91/17428
- DE-A- 4 309 333
- JOURNAL OF PHYSICS D. APPLIED PHYSICS, Bd. 27, Nr. 2, 14.Februar 1994, Seiten 189-193, XP000429783 FANNIN P C ET AL: "ON THE USE OF COMPLEX SUSCEPTIBILITY DATA TO COMPLEMENT MAGNETIC VISCOSITY MEASUREMENTS"
- JOURNAL OF MAGNETISM AND MAGNETIC MATERIALS, Bd. 136, 1994, Seiten 49-58, XP000462355 P.C. FANNIN: "An experimental observation of the dynamic behaviour of ferrofluids"
- MAGNETIC RESONANCE IN MEDICINE, Bd. 31, Nr. 6, Juni 1994, Seiten 611-618, XP000570826 BRONSKILL, M.J. ET AL.: "Analysis of discrete T2 components of NMR relaxation for aqueous solutions in hollow fiber capillaries"
- J. APPLIED PHYSICS, Bd. 49, Nr. 6, 1978, Seiten 3422-3429, XP002005268 BOGARDUS, H. ET AL.: "Dynamic magnetization in ferrofluids"

## Beschreibung

Die Erfindung betrifft den in den Patentansprüchen gekennzeichneten Gegenstand, daß heißt Verfahren zur magnetorelaxometrischen qualitativen und/oder quantitativen Detektion von Analyten in Flüssig- und Festphasen, Verwendung von Verbindungen zur magnetorelaxometrischen Detektion in der Analytik und Immunomagnetographie.

Es ist bekannt, daß die Immunszintigraphie es ermöglicht, pathologische Strukturen *in vivo* mit Hilfe radioaktiv markierter strukturspezifischer Substanzen, die im folgenden auch als Marker bezeichnet werden, zu detektieren. Üblicherweise werden dazu mit γ-Strahlern markierte Antikörper, oder Antikörperfragmente eingesetzt. Daneben kommen auch weitere strukturspezifische Substanzen wie z.B. Peptide oder Oligo- oder Polynukleinsäuren zum Einsatz, bzw. werden in der Forschung erprobt. Der Anteil der spezifisch gebundenen Radioaktiviät ist bei allen diesen Verfahren im allgemeinen jedoch gering. Dementsprechend ist bei diesen Untersuchungen das Niveau der nicht spezifisch gebundenen und damit im Blut zirkulierenden oder sich in Organen wie Leber, Niere, ableitende Harnwege oder Blase akkumulierenden Marker sehr hoch. Diese hohe Hintergrundstrahlung verhindert in vielen Fällen eine ausreichende Detektion der pathologischen Strukturen. Panchapakesan [Immunol. Cell. Biol., 70 (1992) 295 und Ziegler [New England Journal of Medicine, 324 (1991) 430] weisen deshalb auf Verbesserungsmöglichkeiten für die Immunszintigraphie hin. Derartige Möglichkeiten werden auch in der EP 0 251 494 beschrieben. Die meisten der Verfahren zielen darauf ab, die Ausscheidung der nicht spezifisch gebundenen Radioaktivität zu beschleunigen.

Desweiteren wurde verschiedentlich die Verwendung von mit paramagnetischen oder superparamagnetischen Substanzen konjugierten Antikörpern oder Antikörperfragmenten zur Lokalisation pathologischer Strukturen *in vivo* vorgeschlagen. Als Detektionsverfahren kommen bisher für derartige markierte Antikörper die Kernspintomographie oder die auf Suszeptibilitätsänderungen beruhende Magnetometrie (WO 93/05818 und WO 91/15243) in Betracht. Auch bei diesen Detektionsverfahren bleibt das Problem des variablen Signalanteils durch nicht gebundene Anteile des Markers sowie durch natürliche Variationen der Suszeptibilität und Relaxivität des Gewebes vorhanden. Desweiteren sind die Methoden häufig nicht ausreichend empfindlich, um nur geringe Mengen spezifisch gebundener Marker detektieren zu können.

Ein Verfahren, das nur die Detektion des Anteils gebundener Marker ermöglicht und dadurch von dem Ausmaß der nicht gebundenen Marker unbeeinflußt ist, ist dagegen nicht bekannt.

Es ist ferner bekannt, daß quantitative Immunoassays sowie andere Bindungsassays (z.B. Rezeptorbindungsassays) es ermöglichen, eine sehr große Anzahl von Substanzen, die auch von biologischer Relevanz sein können, in Proben unterschiedlicher Zusammensetzung zu bestimmen. In der Regel wird hierbei jedoch nur ein Parameter pro Probe in einem Assay bestimmt. Eine aktuelle Übersicht der verschiedenen Verfahren gibt T. Chard [An Introduction to Radio-immunoassay and Related Techniques: Laboratory Techniques in Biochemistry and Molecular Biology, 4. ed., Elsevier Science Publishers, Amsterdam (1990)]. Grundlage aller Bindungsassays ist die hohe Nachweisempfindlichkeit isotopen- oder anders markierter Verbindungen mit der großen Spezifität von Ligand-Rezeptorreaktionen.

Die bekannten Assayverfahren haben jedoch folgende Nachteile:
- Die Verfahren für die simultane Bestimmung verschiedener Analyten innerhalb derselben Probe basieren auf der Bindung unterschiedlich radio-, fluoreszenz- oder enzymologisch markierter Sonden an die Analyten. Dabei wird in der Regel nach anschließender Separation und Wäsche die nicht gebundene bzw. gebundene Aktivität der Sonde für die quantitative Bestimmung des Analyten gemessen. Die Menge der einsetzbaren unterschiedlichen Sondenlabel ist dabei stark begrenzt. So treten zum Beispiel im Falle von unterschiedlichen Radioisotopen als Sondenmarkierung sogenannte Überlappungsphänomene auf, die zu einem rapiden Verlust der quantitativen Genauigkeit der individuellen Signale führen. Die Kombination verschiedener Enzyme als Sondenlabel verursacht vergleichbare Probleme, wobei die Durchführbarkeit hier zusätzlich durch die erforderliche Suche nach Reaktionsbedingungen, die die simultane Bestimmung der Enzymreaktionen in einem System erlauben, erschwert wird.
- Die Sensitivität der Verfahren ist begrenzt zum Beispiel durch unspezifische Wechselwirkungen zwischen Matrix und Sonde, oder aber durch limitierte Markierungsmöglichkeit der Sonde (geringe spezifische Aktivität).
- Die erfolgreiche Durchführung der Verfahren setzt häufig eine Aufarbeitung des gewonnenen Probenmaterials (z. B. Herstellung von Serum bzw. Plasma aus Vollblut, Extraktion von Proben mit organischen Lösungsmitteln, Anreicherung des Analyten mittels chromatographischer Verfahren u.s.w.) voraus.
- Für die erfolgreiche Durchführung der Verfahren sind Separations- und Waschschritte, die der Trennung von gebundenen und ungebundenen Rezeptoren bzw. Liganden dienen, zumeist unerläßlich.
- Für die Durchführung von Radioimmunoassays ist der Einsatz teurer und in der Handhabung aufwendiger strahlender Nuklide erforderlich.
- Die Lagerung der bisher verwendeten Marker bereitet in der Praxis oft Probleme, da sie entweder nicht stabil sind (Radioimmunoassays) und deshalb stets frisch erzeugt werden müssen oder aber empfindlich auf Umgebungseinflüsse reagieren.

Aufgabe der vorliegenden Erfindung war es daher, neuartige Verfahren zu entwickeln, die die Nachteile des Stand der Technik überwinden und die insbesondere in der Lage sind, ohne Anwendung radioaktiver Substanzen, den Aufenthaltsort und das Ausmaß der gebundenen Marker ohne vorherige Abtrennung von ungebundenem Marker zu detektieren.

Diese Aufgabe wird durch die vorliegende Erfindung gelöst.

Es wurde gefunden, daß die qualitativen und/oder quantitativen Detektion von Analyten in Flüssig- und/oder Festphasen gelingt, wenn ferro- oder ferrimagnetische kolloidale Teilchen als nachzuweisende magnetische Markierung in Immunoassays oder sonstigen Bindungsassays eingesetzt werden und die Relaxation ihrer Magnetisierung als Meßgröße bestimmt wird.

Nachfolgend werden zunächst Verfahren beschrieben, die die Nachteile der bekannten Verfahren zur Durchführung von Immunoassays oder sonstigen Bindungsassays überwinden.

Die erfindungsgemäßen Verfahren beruhen auf der Verwendung kolloidaler ferromagnetischer oder ferrimagnetischer Substanzen, im folgenden auch als magnetische Markierung bezeichnet, die mit nachzuweisenden Substanzen, - im folgenden auch als Analyte bezeichnet -, oder strukturspezifischen Substanzen kombiniert werden. Derartige Kombinationen aus magnetischen Markierungen mit Analyten oder strukturspezifischen Substanzen, die in dieser Patentschrift noch näher beschrieben werden, werden im folgenden auch als magnetische Marker bezeichnet. Durch die Verwendung des Begriffes kolloidale Substanzen oder kolloidale Teilchen wird sowohl der Größenbereich der Teilchen oder Substanzen auf den Größenbereich von Kolloiden, d.h. den Bereich von 1 nm bis ca. 1000 nm, als auch deren Verwendung als dispergierte Phase in einem geeigneten Dispersionsmedium, das zumeist wäßrig ist, beschrieben. Die kolloidalen Substanzen oder Teilchen können zum Zweck der verbesserten Lager- und Transportfähigkeit auch in getrockneter Form oder eingefroren vorliegen, während der Durchführung von Messungen liegen sie jedoch in in flüssiger Phase dispergiertem Zustand vor.

Desweiteren beruhen die Verfahren auf speziellen Meßtechniken, die es erlauben, nach Aufmagnetisierung der magnetischen Markierung, bzw. der magnetischen Marker die Relaxation der Magnetisierung zu bestimmen. Derartige in dieser Patentschrift näher beschriebene erfindungsgemäße Meßverfahren werden im folgenden auch als Magnetrelaxometrie oder Magnetorelaxometrie oder magnetrelaxometrische Detektion bezeichnet.

Eine wesentliche Grundlage der Erfindung ist es, daß die Magnetisierung frei beweglicher ferro- oder ferrimagnetischer kolloidaler Teilchen nach Abschalten eines äußeren magnetisierenden Feldes innerhalb der Meßzeit durch zwei verschiedene Mechanismen relaxiert:
(i) Drehung des gesamten kolloidalen Teilchens innerhalb der umgebenden Flüssigkeit, wobei die Zeitkonstante vom hydrodynamischen Durchmesser der Teilchen einschließlich der Hülle, der Viskosität der Trägerflüssigkeit und der Temperatur abhängt, was hauptsächlich Parameter der Umgebung der Teilchen reflektiert, dieser Mechanismus wird im folgenden auch als Brownsche Relaxation oder extrinsischer Superparamagnetismus bezeichnet und
(ii) Drehung des internen Magnetisierungsvektors innerhalb der kolloidalen Teilchen, wobei die Zeitkonstante sehr empfindlich von Material und Form (Anisotropiekonstante des verwendeten Teilchenmaterials), Volumen und der Temperatur der verwendeten Teilchen abhängt. Dies sind im wesentlichen intrinsische Parameter der Partikel, dieser Mechanismus wird im folgenden auch als Néelsche Relaxation oder intrinsischer Superparamagnetismus bezeichnet.

Die erfindungsgemäße Aufgabe wird dadurch gelöst, daß in Immunoassays oder sonstigen Bindungsassays ferro- oder ferrimagnetische kolloidale Teilchen als nachzuweisende magnetische Markierung eingesetzt werden, deren Brownsche Relaxation unter den Meßbedingungen im ungebundenen Zustand schneller verläuft als die Néelsche Relaxation. Die Anwendung derartiger ferro- oder ferrimagnetischer kolloidaler Teilchen ermöglicht dann, durch die Änderung des dominierenden Relaxationsmechanismus beziehungsweise durch die Vergrößerung des Teilchenvolumens, die durch die Bindung eintritt, die spezifische Bestimmung des Anteils gebundener magnetischer Marker neben gleichzeitig in der Meßprobe vorhandenen ungebundenen magnetischen Markern.

Durch die Verwendung von empfindlichen Meßverfahren können bei erfindungsgemäßem Vorgehen unter Verwendung von ferro- oder ferrimagnetischen kolloidalen Partikeln höchstempfindliche bindungsspezifische Immunoassays oder sonstige Bindungsassays aufgebaut werden, die sowohl in flüssiger Phase als auch an fester Phase durchgeführt werden können. Als besonders empfindliches Meßverfahren kann nach Aufmagnetisierung der Probe in einem magnetisierenden Feld und nach Abschalten des Feldes die Relaxation der Magnetisierung mittels hochempfindlicher Magnetfelddetektoren (wie z.B. Superconducting Quantum Interference Devices (SQUIDs), Induktionsspulen, Fluxgate-Magnetometer, Giant-Magnetoresistance-Sensoren oder magnetoresistive Wandler) oder die komplexe Suszeptibilität der Probe als Funktion der Frequenz des magnetisierenden Feldes bestimmt werden.

Bei den erfindungsgemäßen Verfahren zur magnetrelaxometrischen quantitativen Detektion von Analyten in Flüssig- und Festphasen werden desweiteren die Analyten bindenden strukturspezifischen Substanzen zunächst mit ferrimagnetischen oder ferromagnetischen kolloidalen Teilchen markiert.
Diese magnetisch markierten strukturspezifischen Substanzen werden der zu vermessenden flüssigen oder immobilisierten Probe beigesetzt und die zu vermessende Probe mittels eines von außen angelegten Magnetfeldes aufmagnetisiert Nach Abschalten des äußeren Feldes wird die Relaxation der Magnetisierung der magnetischen Marker mittels Magnetfeldsensoren vermessen.

Die Auswertung der Messergebnisse erfolgt hier, wie auch bei den nachfolgend beschriebenen direkten Assayverfahren, nach dem Fachmann bekannten Methoden.

Das erfindungsgemäße Verfahren zur magnetrelaxometrischen quantitativen Detektion von Analyten in Flüssig- und Festphasen, kann auch so durchgeführt werden, daß Analyte zunächst
(i) mit ferrimagnetischen oder ferromagnetischen kolloidalen Teilchen markiert werden und anschließend
(ii) diese magnetisch markierten Analyte in einer zu vermessenden flüssigen oder immobilisierten Probe eingesetzt werden, der Substanzen zugesetzt werden, die die Analyte spezifisch binden, und die zu vermessende Probe mittels eines von außen angelegten Magnetfeldes aufmagnetisiert wird und nach Abschalten des äußeren Feldes die Relaxation der Magnetisierung der magnetischen Marker mittels Magnetfeldsensoren vermessen wird.

Die Auswertung der Messergebnisse erfolgt hier, wie auch bei den nachfolgend beschriebenen kompetitiven Assayverfahren, in dem Fachmann bekannter Weise, d.h. analog zu den Verfahren wie sie bei Immunoassays oder Radioassays angewandt werden.

In beiden vorgenannten Fällen kann zur Analyse auch die Messung der durch die Bindung veränderten komplexen Suszeptibilität der magnetischen Markierung bzw. des magnetischen Markers als Funktion der Frequenz herangezogen werden.

Die bisher nur in Ausnahmefällen mögliche Diskriminierung zwischen gebundenen und ungebundenen Markern wird durch die Ausnutzung ihrer unterschiedlichen Relaxationsmechanismen oder die durch die Bindung verursachte Beeinflussung der Relaxationszeit des magnetischen Markers ermöglicht.

Festphasengebundene Analyte können erfindungsgemäß insbesondere dadurch nachgewiesen werden, indem die die Analyten bindenden strukturspezifischen Substanzen zunächst
(i) mit im Zeitbereich der Messung relaxierenden ferrimagnetischen oder ferromagnetischen kolloidalen Teilchen markiert werden, wobei die ferrimagnetischen oder ferromagnetischen kolloidalen Teilchen so gewählt werden, daß die Brownsche Relaxation unter den Meßbedingungen eine kürzere Relaxationszeit aufweist als die Néelsche Relaxation und anschließend
(ii) diese magnetisch markierten Substanzen in einer zu vermessenden immobilisierten Probe eingesetzt werden, und die zu vermessende Probe mittels eines von außen angelegten Magnetfeldes geeigneter Stärke aufmagnetisiert wird und nach Abschalten des äußeren Feldes die Relaxation der Magnetisierung der magnetischen Marker mittels Magnetfeldsensoren vermessen wird, wobei das unterschiedliche Relaxationsverhalten festphasengebundener und ungebundener magnetischer Marker zur Analyse herangezogen werden. Als Meßgröße kann auch die komplexe Suszeptibilität der Proben als Funktion der Frequenz bestimmt werden.

Auch in diesem Fall ist es möglich, anstelle strukturspezifischer Substanzen die nachzuweisenden Analyten mit den magnetischen Markierungen zu kombinieren.

In flüssiger Phase können Analyte erfindungsgemäß insbesondere dadurch nachgewiesen werden, indem die die Analyten bindenden strukturspezifischen Substanzen zunächst
(i) mit ferrimagnetischen oder ferromagnetischen kolloidalen Teilchen markiert werden, wobei die ferrimagnetischen oder ferromagnetischen kolloidalen Teilchen so gewählt werden, daß die Brownsche Relaxation unter den Meßbedingungen eine kürzere Relaxationszeit aufweist als die Néelsche Relaxation und anschließend
(ii) diese magnetisch markierten Substanzen in einer zu vermessenden Probe eingesetzt werden, und die zu vermessende Probe mittels eines von außen angelegten Magnetfeldes geeigneter Stärke aufmagnetisiert wird und nach Abschalten des äußeren Feldes die Relaxation der Magnetisierung der magnetischen Marker mittels Magnetfeldsensoren vermessen wird, wobei das unterschiedliche Relaxationsverhalten der mit dem Analyten gebundenen gegenüber den ungebundenen magnetischen Marker zur Analyse herangezogen wird.
   Als Meßgröße kann auch die komplexe Suszeptibilität der Proben als Funktion der Frequenz bestimmt werden.

Auch in diesem Fall ist es möglich, anstelle strukturspezifischer Substanzen die nachzuweisenden Analyten mit den magnetischen Markierungen zu kombinieren.

Unter strukturspezifischen Substanzen sind alle Substanzen zu verstehen, die spezifisch an bestimmte Strukturen binden. Unter strukturspezifischen Substanzen sind insbesondere Antikörper. Antikörperfragmente, Biotin oder Biotin bindende Substanzen wie Avidin und Streptavidin, spezifisch an Rezeptoren bindende Agonisten wie Zytokine, Lymphokine, Endotheline oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate, Lipoproteine etc. zu verstehen. Als strukturspezifische Substanzen sind Substanzen bevorzugt, deren Bindungskonstante im Bereich von 10⁵ - 10¹⁵ (mol/l)⁻¹ liegt. Insbesonders bevorzugt sind Substanzen deren Bindungskonstante im Bereich von 10⁷ - 10¹⁵ (mol/l)⁻¹ liegt.

Die strukturspezifischen Substanzen oder nachzuweisenden Analyte lassen sich mit Hilfe in der Immuno-, Peptid- und Proteinchemie geläufiger Verfahren mit den ferri- oder ferromagnetischen Teilchen markieren. Besonders vorteilhaft sind kovalente Bindungen zwischen den strukturspezifischen Substanzen beziehungsweise den nachzuweisenden Analyten mit den die stabilisierende Hülle der ferri- oder ferromagnetischen Teilchen bildenden Substanzen. Beispiele für besonders geeignete Methoden sind die Aktivierung und Kopplung mittels Carbodiimiden [Jakoby and Wilchek, eds.; Methods Enzymol. (1974) 34], die Bildung von Schiff'schen Basen nach Einwirkung von Periodaten auf Kohlenhydrate enthaltende Verbindungen (Wichek and Bayer, eds., Methods Enzym 184:177), die gegebenenfalls zur weiteren Stabilisierung anschließend noch reduziert werden, die Kopplung mittels Glutardialdehyd [Heitzmann and Richards, Proc. Natl. Acad. Sci. USA 71 (1974) 3537], die Quervernetzung bromoacetylierter Teilchen mit thiolylierten Substanzen [Angerer et al.; Cell 9 (1976) 81], sowie die reduktive Alkylierung (Bayer et al.; J. Histochem. Cytochem. 24 (1976) 933].

Es können auch ferromagnetische oder ferrimagnetische kolloidale Teilchen, mit einer stabilisierenden Hülle aus der strukturspezifischen Substanz oder dem nachzuweisenden Analyten hergestellt werden, indem die Teilchen nach Herstellung direkt in eine Lösung der strukturspezifischen Substanz, gegebenenfalls in Gegenwart weiterer Hilfsstoffe wie z.B. Proteine, Kohlenhydrate sowie natürliche, synthetische oder partialsynthetische oberflächenaktive Substanzen usw. gebracht werden, bzw. direkt in Gegenwart der strukturspezifischen Substanzen hergestellt werden.

Geeignete kolloidale Teilchen und diese Teilchen enthaltende Suspensionen werden beispielsweise in der WO 92/12735, der WO/92/22586, der EP 0 186 616 und der US 4,101,435 beschrieben.

Das erfindungsgemäße Verfahren kann z.B. in der Fertilität, Histokompatibilität, Allergologie, Infektiologie, Hygiene, Genetik, Virologie, Bakteriologie, Toxikologie, Pathologie, Umweltanalytik und medizinischen Diagnostik zum Einsatz kommen.

Ein weiterer Gegenstand der vorliegenden Erfindung ist die Verwendung von Verbindungen zur magnetrelaxometrischen Detektion, die aus kolloidalen Suspensionen frei beweglicher ferrimagnetischer oder ferromagnetischer Teilchen und strukturspezifischen Substanzen beziehungsweise nachzuweisender Analyte bestehen, wobei unter strukturspezifischen Substanzen insbesondere Antikörper, Antikörperfragmente, Biotin oder Biotin bindende Substanzen wie Avidin oder Streptavidin, spezifisch an Rezeptoren bindende Agonisten wie Zytokine, Lymphokine, Endotheline oder deren Antagonisten, sonstige spezifische Peptide und Proteine, Rezeptoren, Enzyme, Enzymsubstrate, Nukleotide, Ribonukleinsäuren, Desoxyribonukleinsäuren, Kohlenhydrate, Lipoproteine etc. zu verstehen sind.

Die Verbindungen zur magnetrelaxometrischen Detektion können auch aus Kombinationen mehrerer ferromagnetischer oder ferrimagnetischer Teilchen mit diskriminierbaren Relaxationszeiten bestehen, da bei Verwendung von unterschiedlichen magnetischen Markierungen mit jeweils sehr enger Verteilung der Relaxationszeiten und/oder magnetischen Momenten für verschiedene strukturspezifische Substanzen, bzw. Analyte innerhalb einer Probe individuell diskriminierbare Meßergebnisse erzielt werden können. Dadurch wird eine direkte simultane quantitative Bestimmung mehrerer Analyte ermöglicht.

Als Suspensionsmedien kommen alle Flüssigkeiten in Betracht, in denen die kolloidalen Teilchen frei beweglich sind. Besonders geeignet sind Wasser, wäßrige Lösungen oberflächenaktiver Hilfsstoffe, wie z.B. Tenside oder oligomere oder polymere Kohlenhydrate und Proteine, sowie Mischungen aus Wasser mit Alkoholen wie z.B. Glycerol und Polyethylenglykol. Die Suspensionsmedien können zusätzlich den osmotischen Druck verändernde Hilfsstoffe wie z.B. Kochsalz enthalten. Desweiteren können den pH-Wert bestimmende Puffersubstanzen, wie z.B. Phosphate, enthalten sein.

Die Verbindungen aus den ferromagnetischen oder ferrimagnetischen kolloidalen Teilchen mit strukturspezifischen Substanzen oder nachzuweisenden Analyten können auch in getrockneter Form, gegebenenfalls in Kombination mit weiteren Hilfsstoffen die z.B. die Trocknung erleichtern oder die Stabilität des getrockneten Produkts erhöhen, vorliegen (z. B. als Lyophilisate).

Die Bestimmung des Analyten kann mit oder ohne Separations- und Waschschritte durchgeführt werden. Bei der Durchführung von Messungen mit Separationsschritten zwischen gebundenen und ungebundenen magnetischen Markern können erfindungsgemäß alle ferro- oder ferrimagnetischen kolloidalen Substanzen als magnetische Markierungen zur magnetrelaxometrischen Detektion eingesetzt werden. Besondere Anforderungen an die Brownschen Relaxationzeiten und die Néelschen Relaxationszeiten sind in diesen Fällen nicht mehr erforderlich.

Aufgrund des auf physikalischen Mechanismen beruhenden Bindungsnachweises können unspezifische Meßsignale (Matrixphänomene) weitgehend ausgeschlossen werden. Die Spezifität des Verfahrens hängt somit nur noch von der "wahren" Spezifität der strukturspezifischen Substanz (Kreuzreaktivität von Antikörpern, unspezifische Bindung von Liganden) ab.
Aufgrund der hohen Sensitivität des erfindungsgemäßen Verfahrens werden die sonst üblichen Detektionsgrenzen von Bindungsassays mühelos unterschritten.

Als Substanzen für die magnetische Markierung können alle ferromagnetischen oder ferrimagnetischen Materialien verwendet werden, die sich in einem zur magnetorelaxometrischen Detektion geeigneten Medium kolloidal dispergieren lassen. Bei Verwendung der Substanzen für magnetorelaxometrische Detektionen, die ohne Separationsschritte zwischen gebundenen und ungebundenen magnetischen Markern durchgeführt werden, muß die Néelsche Relaxationszeit der magnetischen Markierungen unter den Meßbedingungen größer als die Brownsche Relaxationszeit der magnetischen Marker sein. Besonders geeignet sind alle ferromagnetischen oder ferrimagnetischen kolloidalen Teilchen mit Brownschen Relaxationszeiten in wäßrigen Medien im Bereich von 10⁻⁸ - 10⁻¹ s und Néelschen Relaxationszeiten größer als 10⁻⁸ s. Für die Durchführung von Messungen ohne Separationsschritte muß die Viskosität des verwendeten Dispergiermediums mit den Relaxationszeiten der ferromagnetischen und ferrimagnetischen Teilchen und der Meßzeit abgestimmt werden. da das Suspensionsmedium wesentlich die Zeitkonstante der Brownschen Relaxation bestimmt.

Bevorzugt sind insbesondere ferromagnetische oder ferrimagnetische kolloidale Teilchen aus Eisen, Eisenoxiden, Bariumferriten, Strontiumferriten, Kobalt, Nickel, Nickelferriten, Kobaltferriten und Chromdioxid deren Néelsche Relaxationszeit größer als die Brownsche Relaxationszeit ist.

Die Verwendung von magnetischen Markierungen mit eng verteilten Partikelgrößen und/oder magnetischen Momenten ist im allgemeinen vorteilhaft. Eine Auftrennung magnetischer Markierungen in Fraktionen mit enger Verteilung der Partikelgrößen kann z.B. durch chromatographische Verfahren oder unter Verwendung spezieller Filtrationsverfahren (z.B. Glaskapillarsysteme oder Tangentialfiltration), durch die Verwendung von Molekularsieben oder mittels Zentrifugation erreicht werden. Magnetische Markierungen mit möglichst einheitlichen Momenten lassen sich z.B. durch Klassierung in einem magnetischen Gradientenfeld herstelllen.

Die ferromagnetischen und ferrimagnetischen Substanzen können mit einer Hülle aus oligomeren oder polymeren Kohlenhydraten, Proteinen, Peptiden, Nukleotiden, Tensiden, sonstigen Monomeren, Oligomeren oder Polymeren und/ oder Lipiden stabilisiert sein.

Die Teilchengrößen der ferromagnetischen und ferrimagnetischen Substanzen liegen vorteilhafterweise zwischen 1 nm und 400 nm. Insbesonders bevorzugt sind Teilchengrößen zwischen 1 nm und 100 nm.

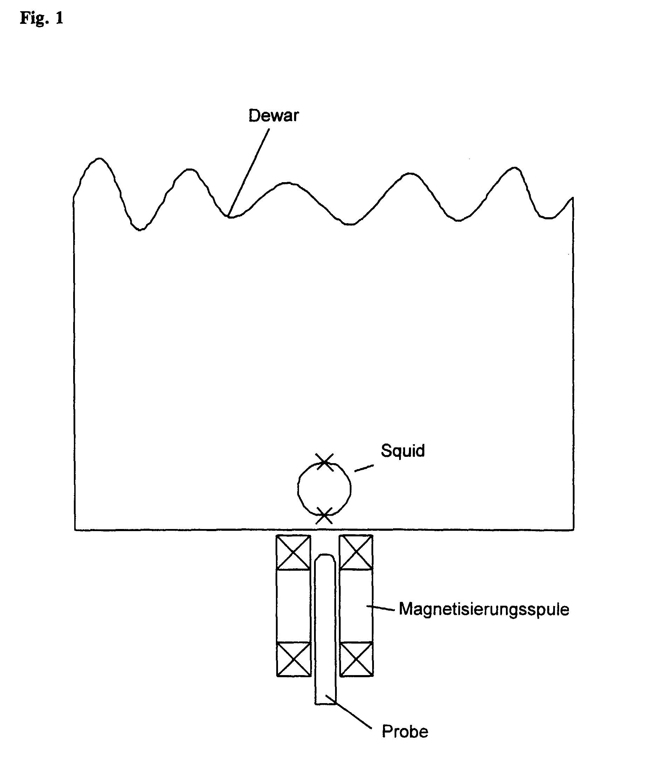

Verfahrensgemäß erfolgt die magnetrelaxometrische Detektion mit Meßanordnungen, die zunächst eine Aufmagnetisierung der zu untersuchenden Probe mittels eines geeigneten Magnetfeldes erlauben und anschließend die Messung der magnetischen Relaxation der magnetischen Marker ermöglichen. Eine Meßanordnung für die magnetrelaxometrische Detektion von Analyten, wie sie in den Beispielen verwendet wurde, ist in Figur 1 dargestellt. Im Gegensatz zu allen anderen bereits bekannten Verfahren (JP-235774 und WO 91/15243) wird bei dem erfindungsgemäßen Verfahren der Messung der Relaxation der Magnetisierung nicht die statische Magnetisierung in Gegenwart des magnetisierenden Feldes gemessen, sondern deren zeitliche Veränderung in Abwesenheit des magnetisierenden Feldes. Erst dadurch werden Informationen über den Bindungszustand der Marker zugänglich. Des weiteren wird dadurch eine Beeinflußung des Meßsignals durch dia- oder paramagnetische Komponenten beziehungsweise Verunreinigungen vermieden. Ferner wird die Meßempfindlichkeit entschieden gesteigert.

Es ist ferner möglich, die Messung der frequenzabhängigen Magnetisierung des Markers infolge eines geeigneten magnetischen Wechselfeldes (Bestimmung der komplexen Suszeptibilität als Funktion der Frequenz) mittels hochempfindlicher Sensoren, wie zum Beispiel SQUIDs, in Anwesenheit des Feldes durchzuführen. Hierbei wird die spezifische Frequenzabhängigkeit des Suszeptibilität des magnetischen Markers im Gegensatz zur separat bestimmbaren Frequenzabhängigkeit der para- oder diamagnetischen Komponenten ausgenutzt. Auch dieses Vorgehen steht im Gegensatz zu dem in WO 91/15243 vorgeschlagenen Verfahren zur Bestimmung der Suszeptibilität superparamagnetischer Substanzen. In WO 91/15243 wird weder die Frequenzabhängigkeit der Suszeptibilität der magnetischen Marker beschrieben, noch ein Verfahren zur Nutzung dieser Eigenschaft.

Ein weiterer Aspekt der Erfindung betrifft Verfahren, die es ermöglichen, den Aufenthaltsort und das Ausmaß der spezifisch gebundenen Marker ohne Beeinflußung durch im Blut zirkulierende Marker zu detektieren. Bei diesen Verfahren wird die Anwendung radioaktiver Substanzen vermieden, wie sie zur Durchführung szintigraphischer Verfahren des Standes der Technik bisher unumgänglich sind.

Die erfindungsgemäßen Verfahren beruhen darauf, daß die Relaxationzeitunterschiede zwischen gebundenen und ungebundenen magnetischen Markern in Flüssigkeiten, sowie die Veränderung des dominierenden Relaxationsmechanismus durch Bindung der magnetischen Marker an Festphasen auch zur magnetrelaxometrischen Detektion von Substanzen oder Strukturen *in vivo* eingesetzt werden können. Derartige Verfahren werden im folgenden auch als Immunmagnetographie oder Immunomagnetographie bezeichnet.

Die *in* vivo-Messung der räumlichen Verteilung einem Menschen applizierter im Zeitbereich der Messung relaxierender magnetischer Marker kann durch zwei verschiedene Meßmethoden erfolgen:
1. Erzeugung eines möglichst homogenen Magnetfeldes im interessierenden Volumen, Abschalten des Feldes und Messung der räumlichen Verteilung des relaxierenden Magnetfeldes mittels eines Multikanalsensors. Dieser sollte das Meßobjekt möglichst vollständig umschließen. Zur Erzeugung ausreichender Meßinformation ist eine mehrfache Meßdurchführung unter sequentieller Abrasterung des Meßobjektes ebenfalls möglich.
2. Sequentielle Erzeugung eines räumlich begrenzten lokalen Feldes, Abschalten des Feldes und Messung der räumlichen Verteilung des relaxierenden Magnetfeldes mittels eines Einkanalsensors. Die Verwendung eines Multikanalsensors ist ebenfalls möglich.

Bei beiden Methoden ist zur Gewinnung maximaler Information sowohl die Magnetisierung des Meßobjektes als auch die Messung des resultierenden Magnetfeldes in allen drei Raumrichtungen zu bevorzugen.

Die Messung läßt sich durch ein Modell beschreiben, wie es auch bei der Analyse der Magnetfelder von bioelektrischen Strömen Anwendung findet. Als Grundlage wird dort das Modell des magnetischen Dipols, Multipols oder Multi-Dipols angenommen. Die speziellen Parameter des Modells, insbesondere die Orte der Dipole oder Multipole, werden durch ein geeignetes Approximationsverfahren gefunden, das die Abweichungen zwischen Meßdaten und Modellparametern minimiert. Diese Parameter geben Aufschluß über die räumliche Verteilung der magnetisierten Teilchen.

Eine analoge Vorgehensweise ist bekannt und bewährt für die Analyse der Magnetfelder von bioelektrischen Strömen.

Als für die Immunmagnetographie geeignete Verfahren und Verbindungen können alle für die magnetrelaxometrische Detektion aufgeführten Verfahren und Substanzen angewendet werden.

Besonders geeignet zur Durchführung der Immunmagnetographie sind magnetische Markierungen, die biologisch abbaubar und verträglich sind. Dies trifft insbesondere für magnetische Markierungen zu, die aus Eisenoxiden bestehen.

Zur Durchführung bindungsspezifischer magnetrelaxometrischer Detektionen *in vivo* ist es erforderlich, daß die Brownschen Relaxationszeiten der in den menschlichen Körper eingebrachten Kombinationen aus ferri- oder ferromagnetischen Substanzen mit strukturspezifischen Substanzen bei Körpertemperatur in Körperflüssigkeiten kürzer als die Néelschen Relaxationszeiten sind.

Unter strukturspezifischen Substanzen sind in der Immunmagnetographie insbesondere alle Substanzen zu verstehen, die spezifisch an nachzuweisende Strukturen des menschlichen Körpers binden. Besonders geeignet sind Antikörper, Antikörperfragmente, spezifisch an Rezeptoren bindende Agonisten oder deren Antagonisten. spezifische Peptide und Proteine, Rezeptoren, Enzyme, Enzymsubstrate, Nukleotide, Ribonukleinsäuren, Desoxyribonukleinsäuren, Kohlenhydrate oder Lipoproteine. Von den an Rezeptoren bindenden Agonisten sind insbesondere Zytokine, Lymphokine oder Endotheline geeignet.

Gut geeignet sind alle strukturspezifischen Substanzen, die eine Bindungskonstante im Bereich von 10⁵- 10¹⁵ (mol/l)⁻¹ aufweisen. Insbesonders geeignet sind alle strukturspezifischen Substanzen, die eine Bindungskonstante im Bereich von 10⁷-10¹⁵ (mol/l)⁻¹ aufweisen.

Die nachfolgenden Beispiele dienen der näheren Erläuterung des Erfindungsgegenstandes, ohne ihn auf diese beschränken zu wollen.

### Beispiel 1

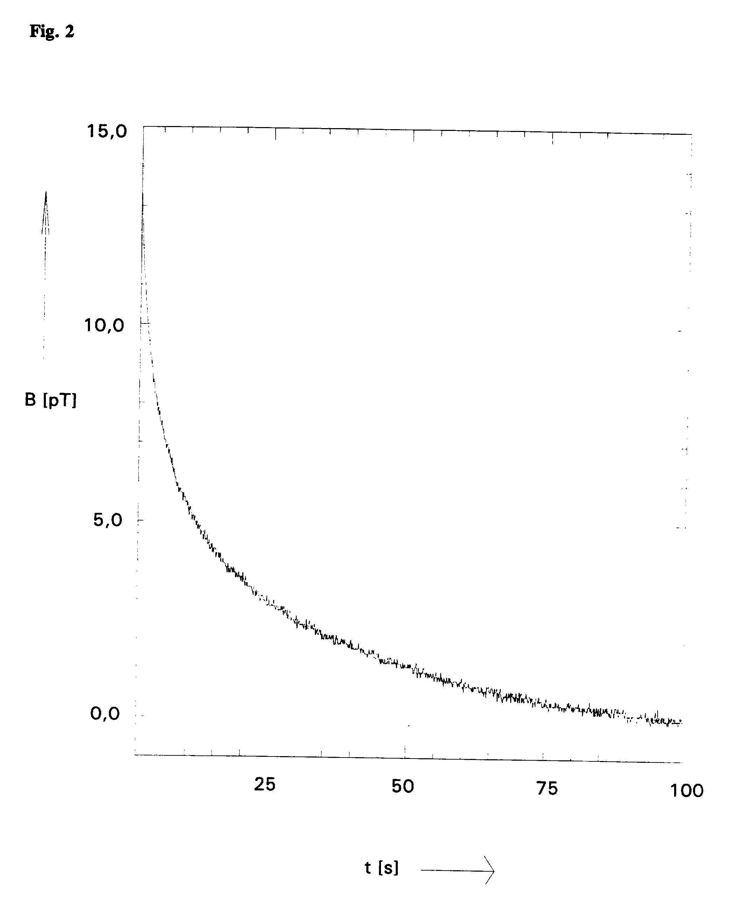

100 µg eines monoklonalen Antikörpers gegen Collagen III, im folgenden Anti-Collagen III genannt, werden in 500 µl 0,1 M Natriumhydrogencarbonat-Lösung gelöst. 1 ml dextrangecoatete Magnetitsuspension (Meito Sangyo) mit 1 Mol Fe/l und einer Teilchengröße von ca. 40 nm wird über eine Sephadex Säule (Pharmacia PD 10) mit 0,1 M Natriumhydrogencarbonat umgepuffert. Zu der Suspension werden 0,5 ml 10 mmol/l Natriumperiodat-Lösung gegeben. Die Lösung wird 2 Stunden im Dunkeln stehengelassen. Anschließend wird über eine PD 10 mit 0,1 M Natriumhydrogencarbonat-Lösung eluiert. Zu der Suspension wird die Anti-Collagen In-Lösung gegeben. Die Mischung wird 3 Stunden bei 4°C im Dunkeln stehengelassen. Anschließend werden 5 mg NaBH₄ als Festsubstanz zugegeben und kurz geschwenkt. Die Mischung wird 8 Stunden bei 4 °C im Dunkeln stehengelassen. Anschließend werden die magnetitmarkierten Anti-Collagen III (im folgenden Mag-Anti-Collagen III genannt) über eine PD 10 Säule mit phosphatgepufferter Kochsalzlösung (nachfolgend PBS, pH 7,4) eluiert. Eine Lösung von 5 µg Collagen III in 200 µl Puffer (phosphatgepufferte Kochsalzlösung (PBS)) wird in einem Probengefäß aus Polystyrol inkubiert. Anschließend wird die Flüssigphase verworfen. Das Probengefäß wird dreimal mit phosphatgepufferte Kochsalzlösung, enthaltend 0,1 % Tween® 20 (nachfolgend PBST genannt), gespült. Zu der Probe werden 5 µl Mag-Anti-Collagen III in 200 µl PBST gegeben. Es wird 1 Stunde bei Raumtemperatur inkubiert. Anschließend wird die Probe in einer magnetisch abgeschirmten Kammer in einem Feld der Stärke 2 mT 4 cm unterhalb des Squid-Detektors magnetisiert (siehe Figur 1). 400 Millisekunden nach Abschalten des Magnetfeldes wird über 100 Sekunden die Relaxationsmessung durchgeführt. Bei der Probe wird über ein abklingendes Feld eine Relaxation nachgewiesen. Das Relaxationssignal der Collagen III enthaltenden Probe ist in Figur 2 dargestellt.

### Beispiel 2

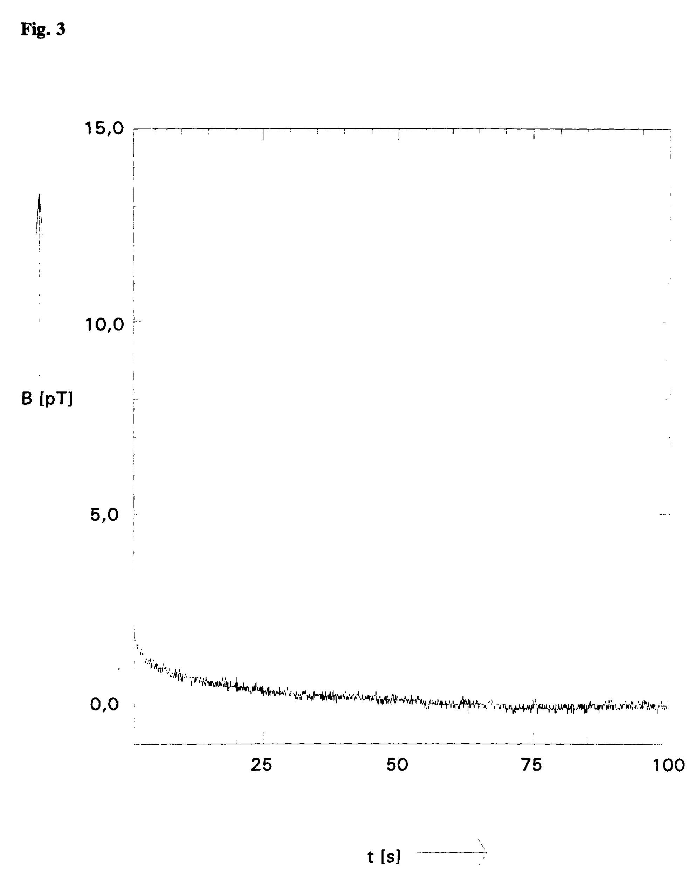

Eine Lösung von 5 µg Collagen V in 200 µl PBS Puffer pH 7,4 wird in einem Probengefäß aus Polystyrol inkubiert. Anschließend wird die Flüssigphase verworfen. Das Probengefäß wird dreimal mit PBST Waschpuffer pH 7,4 gespült. Zu der Probe werden 5 µl Mag-Anti-Collagen III, hergestellt nach Beispiel 1, in 200 µl PBST gegeben. Es wird 1 Stunde bei Raumtemperatur inkubiert. Anschließend wird die Probe in einer magnetisch abgeschirmten Kammer in einem Feld der Stärke 2 mT 4 cm unterhalb des SQUID-Detektors magnetisiert (siehe Figur 1). Nach Abschalten des Magnetisierungsfeldes wird die Probe vermessen. 400 Millisekunden nach Abschalten des Magnetfeldes wird über 100 Sekunden die Relaxationsmessung durchgeführt. Bei der Collagen V enthaltenden Probe ist im Rahmen der Meßsicherheit kein abklingendes magnetisches Feld detektierbar (siehe Figur 3).

### Beispiel 3

Zu einer Lösung von 100 µg Collagen III in 1 ml PBS werden 100 µl Glutardialdehydlösung (3 % in Wasser) gegeben. Die Lösung wird 24 h bei 4°C gerührt und danach abzentrifugiert. Das Pellet enthält ausgefallenes vernetztes Collagen III. Das vernetzte Collagen III wird in 1 ml PBS suspendiert. (Probe 1). Zu einer Lösung von 100 µg Collagen V in 1 ml PBS werden 100 µl Glutardialdehydlösung (3 % in Wasser) gegeben. Die Lösung wird 24 h bei 4°C gerührt und danach abzentrifugiert. Das Pellet enthält ausgefallenes vernetztes Collagen V. Das vernetzte Collagen V wird in 1 ml PBS suspendiert. (Probe 2). Zu den Proben 1 und 2 werden je 5 µl der Mag-Anti-Collagen III-Suspension aus Beispiel 1 gegeben. Es wird 1 h bei 37°C inkubiert. Anschließend werden beide Proben in einer abgeschirmten Kammer in einem Magnetfeld der Stärke 2 mT unter einem SQUID-Detektor magnetisiert. 400 Millisekunden nach Abschalten des Magnetisierungsfeldes wird die Relaxationsmessung durchgeführt. Bei Probe 1 wird ein abklingendes Feld gemessen. Bei Probe 2 ist kein abklingendes Feld detektierbar.

### Beispiel 4

Aus 10 ml einer 1,9 mg/ml Collagen III-Lösung in PBS (pH 7,4) werden jeweils 5 ml der folgenden Verdünnungen hergestellt:
10000ng/ml, 1000 ng/ml, 100 ng/ml, 10 ng/ml, 1 ng/ml

Von jeder Verdünnung werden je drei mal 1 ml in Polystyrolröhrchen (Fassungsvolumen 2,5 ml) pipettiert. Es wird 1 Stunde bei 37°C inkubiert. Danach wird der Inhalt der Röhrchen verworfen. Die Röhrchen werden 3mal mit je 1 ml PBST gewaschen.

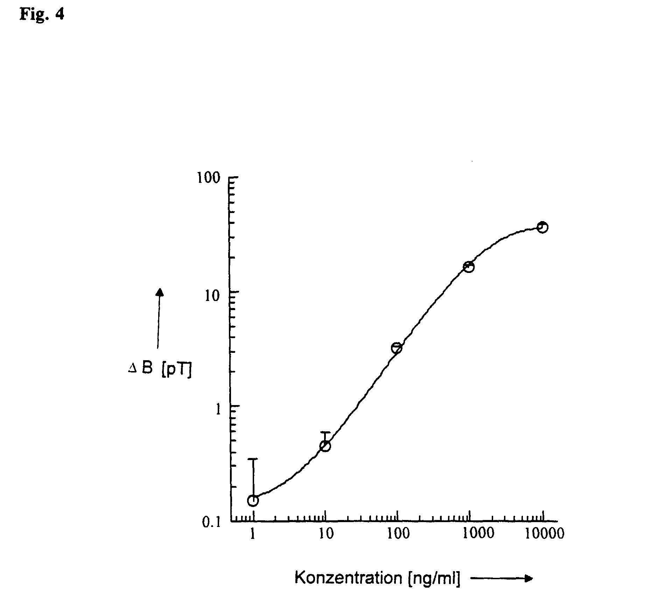

In jedes Röhrchen werden 1 ml einer 1 : 100-Verdünnung des Magnetit markierten Antikörpers, hergestellt gemäß Beispiel 1, gegeben. Die Röhrchen werden 1 Stunde bei Raumtemperatur stehengelassen. Danach werden die Proben mit der in Figur 1 skizzierten Meßanordnung magnetisiert (2 mT) und nach Abschalten des magnetisierenden Feldes wird über 100 Sekunden die Relaxation gemessen. Die Auswertung der Differenzen der gemessenen magnetischen Flußdichten B, 200 Millisekunden und 100 Sekunden nach Abschalten des magnetisierenden Feldes, in Abhängigkeit von der Collagenkonzentration in der Probe, ist in Figur 4 wiedergegebenen.

### Beispiel 5

100 µg eines monoklonalen Antikörpers gegen Collagen III, im folgenden Anti-Collagen III genannt, werden in 500 µl 0,1 M Natriumhydrogencarbonat-Lösung gelöst. 1 ml dextrangecoatete Magnetitsuspension mit 1 Mol Fe/l und einer Teilchengröße von ca. 40 nm wird über eine Sephadex Säule (Pharmacia PD 10) mit 0,1 M Natriumhydrogencarbonat umgepuffert. Zu der Suspension werden 0,5 ml 10 mmol/l Natriumperiodat-Lösung gegeben. Die Lösung wird 2 Stunden im Dunkeln stehengelassen. Anschließend wird über eine PD 10 mit 0,1 M Natriumhydrogencarbonat-Lösung eluiert. Zu der Suspension wird die Anti-Collagen III-Lösung gegeben. Die Mischung wird 3 Stunden bei 4°C im Dunkeln stehengelassen. Anschließend werden 5 mg NaBH₄ als Festsubstanz zugegeben und kurz geschwenkt. Die Mischung wird 8 Stunden bei 4 °C im Dunkeln stehengelassen. Anschließend werden die magnetitmarkierten Anti-Collagen III (im folgenden Mag-Anti-Collagen III genannt) über eine PD 10 Säule mit phosphatgepufferter Kochsalzlösung (PBS, pH 7,4) eluiert.
Je 20 µl der Mag-Anti-Collagen III-Suspension werden mit 390 µl phosphatgepufferter Kochsalzlösung pH 7,4, die zusätzlich 0,1 % PBST enthält verdünnt und in drei Probengefäße aus Polyacrylsäure gefüllt, die jeweils ein Fassungsvolumen von 500 µl aufweisen. Zum ersten Probengefäß werden 100 µl einer wäßrigen Lösung von humanem Serumalbumin (1 mg Albumin / ml) gegeben (Probe 1). Zum zweiten Probengefäß werden 100 µl einer Lösung von Collagen V in PBST (1 µg Collagen V / ml) gegeben (Probe 2). Zum dritten Probengefäß werden 100 µl einer Lösung von Collagen III in PBST (1 µg Collagen III / ml) gegeben (Probe 3). 200 Sekunden nach Zugabe der Proteinlösungen werden die Proben mit der in Figur 1 skizzierten Meßanordnung magnetisiert (2 mT) und 20 Millisekunden nach Abschalten des magnetisierenden Feldes beginnend über 1 Sekunden jeweils die magnetische Relaxation bestimmt. In den Proben 1 und 2 ist im Rahmen der Meßsicherheit kein abklingendes magnetisches Feld detektierbar. In der Probe 3 ist dagegen ein abklingendes magnetisches Feld detektierbar. Die Messungen werden nach Entleeren der Probengefäße und dreimaligem Spülen mit je 500 µl PBST wiederholt. Es ist jetzt in keinem der Probengefäße im Rahmen der Meßsicherheit ein abklingendes magnetisches Feld detektierbar.

### Beispiel 6

100 µg Avidin werden in 500 µl 0,1 M Natriumhydrogencarbonat-Lösung gelöst. 1 ml dextrangecoatete Magnetitsuspension mit 1 Mol Fe/l und einer Teilchengröße von ca. 40 nm wird über eine Sephadex Säule (Pharmacia PD 10) mit 0,1 M Natriumhydrogencarbonat umgepuffert. Zu der Suspension werden 0,5 ml 10 mmol/l Natriumperiodat-Lösung gegeben. Die Lösung wird 2 Stunden im Dunkeln stehengelassen. Anschließend wird über eine PD 10 mit 0,1 M Natriumhydrogencarbonat-Lösung eluiert. Zu der Suspension wird die Avidin-Lösung gegeben. Die Mischung wird 3 Stunden bei 4°C im Dunkeln stehengelassen. Anschließend werden 5 mg NaBH₄ als Festsubstanz zugegeben und kurz geschwenkt. Die Mischung wird 8 Stunden bei 4 °C im Dunkeln stehengelassen. Anschließend werden das magnetitmarkierte Avidin (im folgenden Mag-Avidin genannt) über eine PD 10 Säule mit phosphatgepufferter Kochsalzlösung (PBS, pH 7,4) eluiert.
1 mg Rinderserumalbumin wird mit Biotin-N-Hydroxysuccinimid (Sigma) konjugiert (im folgenden als Biotin-Albumin bezeichnet) und zu einer Konzentration von 1 µg / ml in PBS verdünnt.
1 ml der Biotin-Albumin-Verdünnung wird 3 h bei Raumtemperatur in einem Probengefäß aus Polystyrol inkubiert. Anschließend wird die Flüssigphase verworfen. Das Probengefäß wird dreimal mit phosphatgepufferter Kochsalzlösung, enthaltend 0,1 % Tween® 20 (PBST), gespült. Zu der Probe werden 5 µl Mag-Avidin gegeben. Es wird 1 Stunde bei Raumtemperatur inkubiert. Anschließend wird die Probe in einer magnetisch abgeschirmten Kammer in einem Feld der Stärke 2 mT 4 cm unterhalb des Squid-Detektors magnetisiert (siehe Figur 1). 400 Millisekunden nach Abschalten des Magnetfeldes wird über 100 Sekunden die Relaxationsmessung durchgeführt. Bei der Probe wird ein abklingendes magnetisches Feld gemessen.
1 ml einer Verdünnung von Rinderserumalbumin in PBS (0,1 mg / ml) wird 3 h bei Raumtemperatur in einem Probengefäß aus Polystyrol inkubiert. Anschließend wird die Flüssigphase verworfen. Das Probengefäß wird dreimal mit phosphatgepufferte
Kochsalzlösung, enthaltend 0,1 % Tween® 20 (PBST), gespült. Zu der Probe werden 5 µl Mag-Avidin gegeben. Es wird 1 Stunde bei Raumtemperatur inkubiert. Anschließend wird die Probe in einer magnetisch abgeschirmten Kammer in einem Feld der Stärke 2 mT 4 cm unterhalb des Squid-Detektors magnetisiert (siehe Figur 1). 400 Millisekunden nach Abschalten des Magnetfeldes wird über 100 Sekunden die Relaxationsmessung durchgeführt. Bei der Probe wird im Rahmen der Meßsicherheit kein abklingendes magnetisches Feld gemessen.

## Patentansprüche

1. Verfahren zur qualitativen und/oder quantitativen Detektion von Analyten in Flüssig- und/oder Festphasen dadurch gekennzeichnet, daß ferro- oder ferrimagnetische kolloidale Teilchen als nachzuweisende magnetische Markierung in Immunoassays oder sonstigen Bindungsassays eingesetzt werden und die Relaxation ihrer Magnetisierung als Meßgröße bestimmt wird.

2. Verfahren zur quantitativen Detektion von Analyten in Flüssig- und Festphasen mittels magnetischer Relaxationsmessungen, dadurch gekennzeichnet, daß ferro- oder ferrimagnetische kolloidale Teilchen als nachzuweisende magnetische Markierung in Immunoassays oder sonstigen Bindungsassays eingesetzt werden, deren Brownsche Relaxation unter den Meßbedingungen schneller verläuft als die Néelsche Relaxation.

3. Verfahren zur quantitativen Detektion von Analyten in Flüssig- und Festphasen mittels Messung der komplexen Suszeptibilität als Funktion der Frequenz dadurch gekennzeichnet, daß ferro- oder ferrimagnetische kolloidale Teilchen als nachzuweisende magnetische Markierung in Immunoassays oder sonstigen Bindungsassays eingesetzt werden, deren Brownsche Relaxation unter den Meßbedingungen schneller verläuft als die Néelsche Relaxation.

4. Verfahren zur quantitativen Detektion von Analyten in Flüssig- und Festphasen, dadurch gekennzeichnet, daß die die Analyten bindenden strukturspezifischen Substanzen zunächst
(i) mit ferrimagnetischen oder ferromagnetischen kolloidalen Teilchen markiert werden und anschließend
(ii) diese markierten strukturspezifischen Substanzen in einer zu vermessenden flüssigen oder immobilisierten Probe eingesetzt werden, die zu vermessende Probe mittels eines von außen angelegten Magnetfeldes aufmagnetisiert wird und nach Abschalten des äußeren Feldes die Relaxation der Magnetisierung der magnetischen Marker mittels Magnetfeldsensoren vermessen wird.

5. Verfahren zur quantitativen Detektion von Analyten in Flüssig- und Festphasen, dadurch gekennzeichnet, daß Analyte zunächst
(i) mit ferrimagnetischen oder ferromagnetischen kolloidalen Teilchen markiert werden und anschließend
(ii) diese magnetisch markierten Analyte in einer zu vermessenden flüssigen oder immobilisierten Probe eingesetzt werden, der Substanzen zugesetzt wurden, die die Analyte spezifisch binden, und die zu vermessende Probe mittels eines von außen angelegten Magnetfeldes aufmagnetisiert wird und nach Abschalten des äußeren Feldes die Relaxation der Magnetisierung der magnetischen Marker mittels Magnetfeldsensoren vermessen wird.

6. Verfahren zur quantitativen Detektion von festphasengebundenen Analyten, dadurch gekennzeichnet, daß die die Analyten bindenden strukturspezifischen Substanzen zunächst
(i) mit im Zeitbereich der Messung relaxierenden ferrimagnetischen oder ferromagnetischen kolloidalen Teilchen markiert werden, wobei die ferrimagnetischen oder ferromagnetischen kolloidalen Teilchen so gewählt werden, daß die Brownsche Relaxation unter den Meßbedingungen eine kürzere Relaxationszeit aufweist als die Néelsche Relaxation und anschließend
(ii) diese magnetisch markierten Substanzen in einer zu vermessenden immobilisierten Probe eingesetzt werden, und die zu vermessende Probe mittels eines von außen angelegten Magnetfeldes aufmagnetisiert wird und nach Abschalten des äußeren Feldes die Relaxation der Magnetisierung der magnetischen Marker mittels Magnetfeldsensoren vermessen wird, wobei das unterschiedliche Relaxationsverhalten der mit dem Analyten gebundenen gegenüber den ungebundenen magnetischen Markern zur Analyse herangezogen wird.

7. Verfahren nach Anspruch 6, dadurch gekennzeichnet, daß nachzuweisende Analyte mit im Zeitbereich der Messung relaxierenden ferrimagnetischen oder ferromagnetischen kolloidalen Teilchen markiert werden, deren Brownsche Relaxation unter den Meßbedingungen eine kürzere Relaxationszeit aufweist als die Néelsche Relaxation und den zu vermessenden Proben zusätzlich die Analyte spezifisch bindende Substanzen zugesetzt werden, beziehungsweise die Analyte spezifisch bindende Substanzen in der Probe immobilisiert vorliegen.

8. Verfahren zur quantitativen Detektion von in flüssiger Phase vorliegenden Analyten, dadurch gekennzeichnet, daß die die Analyten bindenden strukturspezifischen Substanzen zunächst
(i) mit ferrimagnetischen oder ferromagnetischen kolloidalen Teilchen markiert werden, deren Brownsche Relaxation unter den Meßbedingungen eine kürzere Relaxationszeit aufweist als die Néelsche Relaxation und anschließend
(ii) diese magnetisch markierten Substanzen in einer zu vermessenden Probe eingesetzt werden, und die zu vermessende Probe mittels eines von außen angelegten Magnetfeldes aufmagnetisiert wird und nach Abschalten des äußeren Feldes die Relaxation der Magnetisierung der magnetischen Marker mittels Magnetfeldsensoren vermessen wird, wobei das unterschiedliche Relaxationsverhalten der mit dem Analyten gebundenen gegenüber den ungebundenen magnetischen Markern zur Analyse herangezogen wird.

9. Verfahren nach Anspruch 8, dadurch gekennzeichnet, daß nachzuweisende Analyte mit ferrimagnetischen oder ferromagnetischen kolloidalen Teilchen markiert werden, deren Brownsche Relaxation unter den Meßbedingungen eine kürzere Relaxationszeit aufweist als die Néelsche Relaxation und den zu vermessenden Proben zusätzlich die Analyte spezifisch bindende Substanzen zugesetzt wurden.

10. Verfahren gemäß den Ansprüchen 1 und 4 bis 9, dadurch gekennzeichnet, daß zur Detektion die Messung der komplexen Suszeptibilität als Funktion der Frequenz herangezogen wird.

11. Verfahren gemäß den Ansprüchen 1 bis 10, dadurch gekennzeichnet, daß die strukturspezifischen Substanzen Antikörper, Antikörperfragmente, Biotin oder Biotin spezifisch bindende Substanzen wie Avidin oder Streptavidin, spezifisch an Rezeptoren bindende Agonisten oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate oder Lipoproteine sind.

12. Verfahren gemäß Anspruch 11, dadurch gekennzeichnet, daß die an Rezeptoren bindenden Agonisten Zytokine, Lymphokine oder Endotheline sind.

13. Verfahren gemäß den Ansprüchen 11 und 12, dadurch gekennzeichnet, daß die strukturspezifischen Substanzen eine Bindungskonstante im Bereich von 105 - 10¹⁵ (mol/l)⁻¹ haben.

14. Verfahren gemäß den Ansprüchen 11 und 12, dadurch gekennzeichnet, daß die strukturspezifischen Substanzen eine Bindungskonstante im Bereich von 10⁷ - 10¹⁵ (mol/l)⁻¹ haben.

15. Verfahren gemäß den Ansprüchen 1 bis 14, dadurch gekennzeichnet, daß als Magnetfeldsensoren Superconducting Quantum Interference Devices (SQUIDs), Induktionsspulen, Fluxgate-Magnetometer, Giant-Magnetoresistance-Sensoren oder magnetoresistive Wandler eingesetzt werden.

16. Verfahren gemäß den Ansprüchen 1 bis 15, dadurch gekennzeichnet, daß eine Bestimmung von zwei oder mehreren verschiedenen Analyten in einer Probe durchgeführt wird.

17. Verfahren gemäß Anspruch 16, dadurch gekennzeichnet, daß zwei oder mehrere ferromagnetische oder ferrimagnetische Substanzen mit diskriminierbaren Brownschen oder Néelschen Relaxationszeiten verwendet werden.

18. Verwendung von Verbindungen zur quantitativen Detektion von Analyten in Flüssig- und Festphasen mittels magnetischer Relaxationsmessungen oder mittels Messungen der komplexen Suszeptibilität als Funktion der Frequenz, dadurch gekennzeichnet, daß sie aus Kombinationen von ferro- oder ferrimagnetischen kolloidalen Teilchen mit strukturspezifischen Substanzen oder nachzuweisenden Substanzen bestehen, deren Brownsche Relaxation unter den Meßbedingungen schneller verläuft als die Néelsche Relaxation.

19. Verfahren gemäß den Ansprüchen 1 bis 17, dadurch gekennzeichnet, daß die ferromagnetischen und ferrimagnetischen Substanzen eine Teilchengröße im Bereich von 1 bis 400 nm haben.

20. Verfahren gemäß den Ansprüchen 1 bis 17, dadurch gekennzeichnet, daß die ferromagnetischen und ferrimagnetischen Substanzen eine Teilchengröße im Bereich von 1 bis 100 nm haben.

21. Verfahren gemäß den Ansprüchen 1 bis 17, dadurch gekennzeichnet, daß die ferromagnetischen und ferrimagnetischen Substanzen mit einer Hülle aus oligomeren oder polymeren Kohlenhydraten, Proteinen, Peptiden, Nukleotiden und/ oder Lipiden stabilisiert sind.

22. Verwendung gemäß Anspruch 18, dadurch gekennzeichnet, daß die ferromagnetischen und ferrimagnetischen Substanzen eine Teilchengröße im Bereich von 1 bis 400 nm haben.

23. Verwendung gemäß Anspruch 18, dadurch gekennzeichnet, daß die ferromagnetischen und ferrimagnetischen Substanzen eine Teilchengröße im Bereich von 1 bis 100 nm haben.

24. Verwendung gemäß den Ansprüchen 18, 22 und 23, dadurch gekennzeichnet, daß die ferromagnetischen und ferrimagnetischen Substanzen mit einer Hülle aus oligomeren oder polymeren Kohlenhydraten, Proteinen, Peptiden, Nukleotiden, Tensiden, Polymeren und/ oder Lipiden stabilisiert sind.

25. Verwendung gemäß den Ansprüchen 18, 22 und 23, dadurch gekennzeichnet, daß die strukturspezifischen Substanzen Antikörper, Antikörperfragmente, Biotin oder Biotin bindende Substanzen wie Avidin oder Streptavidin, spezifisch an Rezeptoren bindende Agonisten oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate, oder Lipoproteine sind.

26. Verwendung der Verfahren gemäß den Ansprüchen 1 - 17 und 19 bis 21 in der Fertilität, Histokompatibilität, Allergologie, Infektiologie, Hygiene, Genetik, Virologie, Bakteriologie, Toxikologie, Pathologie, Umweltanalytik und medizinischen Diagnostik.

27. Verwendung der Verbindungen gemäß den Ansprüchen 18, 22 und 23 in der Fertilität, Histokompatibilität, Allergologie, Infektiologie, Hygiene, Genetik, Virologie, Bakteriologie, Toxikologie, Pathologie, Umweltanalytik und medizinischen Diagnostik.

28. Verwendung von Kombinationen aus ferri- oder ferromagnetischen Substanzen mit strukturspezifischen Substanzen oder von Kombinationen aus ferri- oder ferromagnetischen Substanzen mit nachzuweisenden Analyten in Verfahren gemäß den Ansprüchen 1 - 17 und 19 bis 21.

29. Verwendung von Verbindungen zur Anwendung in Verfahren gemäß den Ansprüchen 1 - 17 und 19 - 21, dadurch gekennzeichnet, daß sie aus Kombinationen biologisch abbaubarer ferri- oder ferromagnetischer Substanzen mit strukturspezifischen Substanzen bestehen, wobei die Brownschen Relaxationszeiten der Kombinationen aus ferri- oder ferromagnetischen Substanzen mit strukturspezifischen Substanzen bei Körpertemperatur in Körperflüssigkeiten kürzer als die Néelschen Relaxationszeiten sind.

30. Verwendung gemäß Anspruch 29, dadurch gekennzeichnet, daß die ferri- oder ferromagnetischen Substanzen Eisenoxide sind, die biologisch abbaubar sind.

## Claims

1. Method for the qualitative and/or quantitative detection of analytes in liquid and/or solid phases, characterized in that ferro- or ferrimagnetic colloidal particles are employed as magnetic label to be detected in immumoassays or other binding assays, and the relaxation of their magnetization is determined as measured variable.

2. Method for the quantitative detection of analytes in liquid and solid phases by means of magnetic relaxation measurements, characterized in that ferro- or ferrimagnetic colloidal particles are employed as magnetic label to be detected in immumoassays or other binding assays, the brownian relaxation thereof taking place more quickly than the Néel-type relaxation under the measurement conditions.

3. Method for the quantitative detection of analytes in liquid and solid phases by measuring the complex susceptibility as a function of the frequency, characterized in that ferro- or ferrimagnetic colloidal particles are employed as magnetic label to be detected in immumoassays or other binding assays, the brownian relaxation thereof taking place more quickly than the Néel-type relaxation under the measurement conditions.

4. Method for the quantitative detection of analytes in liquid and solid phases, characterized in that firstly the structure-specific substances binding the analytes
(i) are labelled with ferrimagnetic or ferromagnetic colloidal particles and then
(ii) these labelled structure-specific substances are employed in a liquid or immobilized sample to be measured, and the sample to be measured is magnetized using an externally imposed magnetic field and, after the external field has been switched off, the relaxation of the magnetization of the magnetic markers is measured using magnetic field sensors.

5. Method for the quantitative detection of analytes in liquid and solid phases, characterized in that firstly analytes
(i) are labelled with ferrimagnetic or ferromagnetic colloidal particles and then
(ii) these magnetically labelled analytes are employed in a liquid or immobilized sample to be measured, to which have been added substances which specifically bind the analytes, and the sample to be measured is magnetized using an externally imposed magnetic field and, after the external field has been switched off, the relaxation of the magnetization of the magnetic markers is measured using magnetic field sensors.

6. Method for the quantitative detection of solid phase-bound analytes, characterized in that firstly the structure-specific substances binding the analytes
(i) are labelled with ferrimagnetic or ferromagnetic colloidal particles which relax in the measurement time interval, with the ferrimagnetic or ferromagnetic colloidal particles being chosen so that the brownian relaxation shows a shorter relaxation time than the Néel-type relaxation under the measurement conditions, and then
(ii) these magnetically labelled substances are employed in an immobilized sample to be measured, and the sample to be measured is magnetized using an externally imposed magnetic field and, after the external field has been switched off, the relaxation of the magnetization of the magnetic markers is measured by magnetic field sensors, using the difference in relaxation behaviour between analyte-bound and unbound magnetic markers for the analysis.

7. Method according to Claim 6, characterized in that analytes to be detected are labelled with ferrimagnetic or ferromagnetic colloidal particles which relax in the measurement time interval and whose brownian relaxation shows a shorter relaxation time than the Néel-type relaxation under the measurement conditions, and in addition substances specifically binding the analytes are added to the samples to be measured, or substances specifically binding the analytes are immobilized in the sample.

8. Method for the quantitative detection of analytes present in liquid phase, characterized in that firstly the structure-specific substances binding the analytes
(i) are labelled with ferrimagnetic or ferromagnetic colloidal particles, whose brownian relaxation shows a shorter relaxation time than the Néel-type relaxation under the measurement conditions and
(ii) these magnetically labelled substances are employed in a sample to be measured, and the sample to be measured is magnetized using an externally imposed magnetic field and, after the external field has been switched off, the relaxation of the magnetization of the magnetic markers is measured by magnetic field sensors, using the difference in relaxation behaviour between the analyte-bound and unbound magnetic markers for the analysis.

9. Method according to Claim 8, characterized in that analytes to be detected are labelled with ferrimagnetic or ferromagnetic colloidal particles whose brownian relaxation shows a shorter relaxation time than the Néel-type relaxation under the measurement conditions, and in addition substances specifically binding the analytes have been added to the samples to be measured.

10. Method according to Claims 1 and 4 to 9, characterized in that measurement of the complex susceptibility as a function of the frequency is used for the detection.

11. Method according to Claims 1 to 10, characterized in that the structure-specific substances are antibodies, antibody fragments, biotin or substances specifically binding biotin, such as avidin or streptavidin, agonists specifically binding to receptors or antagonists thereof, specific peptides and proteins, receptors, enzymes, enzyme substrates, nucleotides, ribonucleic acids, deoxyribonucleic acids, carbohydrates or lipoproteins.

12. Method according to Claim 11, characterized in that the agonists binding to receptors are cytokines, lymphokines or endothelins.

13. Method according to Claims 11 and 12, characterized in that the structure-specific substances have a binding constant in the range 10⁵ - 10¹⁵ (mol/l)⁻¹.

14. Method according to Claims 11 and 12, characterized in that the structure-specific substances have a binding constant in the range 10⁷ - 10¹⁵ (mol/l)⁻¹.

15. Method according to Claims 1 to 14, characterized in that the magnetic field sensors employed are superconducting quantum interference devices (SQUIDS), induction coils, fluxgate magnetometers, giant magnetoresistance sensors or magnetoresistive tranducers.

16. Method according to Claims 1 to 15, characterized in that a determination of two or more different analytes is carried out in one sample.

17. Method according to Claim 16, characterized in that two or more ferromagnetic or ferrimagnetic substances with brownian or Néel-type relaxation times which can be discriminated are used.

18. Use of compounds for the quantitative detection of analytes in liquid and solid phases by magnetic relaxation measurements or by measurements of the complex susceptibility as a function of the frequency, characterized in that they consist of combinations of ferro- or ferrimagnetic colloidal particles with structure-specific substances or substances to be detected, the brownian relaxation thereof taking place faster than the Néel-type relaxation under the measurement conditions.

19. Method according to Claims 1 to 17, characterized in that the ferromagnetic and ferrimagnetic substances have a particle size in the range from 1 to 400 nm.

20. Method according to Claims 1 to 17, characterized in that the ferromagnetic and ferrimagnetic substances have a particle size in the range from 1 to 100 nm.

21. Method according to Claims 1 to 17, characterized in that the ferromagnetic and ferrimagnetic substances are stabilized with an envelope of oligomeric or polymeric carbohydrates, proteins, peptides, nucleotides and/or lipids.

22. Use according to Claim 18, characterized in that the ferromagnetic and ferrimagnetic substances have a particle size in the range from 1 to 400 nm.

23. Use according to Claim 18, characterized in that the ferromagnetic and ferrimagnetic substances have a particle size in the range from 1 to 100 nm.

24. Use according to Claims 18, 22 and 23, characterized in that the ferromagnetic and ferrimagnetic substances are stabilized with an envelope of oligomeric or polymeric carbohydrates, proteins, peptides, nucleotides, surfactants, polymers and/or lipids.

25. Use according to Claims 18, 22 and 23, characterized in that the structure-specific substances are antibodies, antibody fragments, biotin or substances binding biotin, such as avidin or streptavidin, agonists specifically binding to receptors or antagonists thereof, specific peptides and proteins, receptors, enzymes, enzyme substrates, nucleotides, ribonucleic acids, deoxyribonucleic acids, carbohydrates or lipoproteins.

26. Use of the methods according to Claims 1-17 and 19 to 21 in fertility, histocompatibility, allergology, infectiology, hygiene, genetics, virology, bacteriology, toxicology, pathology, environmental analysis and medical diagnosis.

27. Use of the compounds according to Claims 18, 22 and 23 in fertility, histocompatibility, allergology, infectiology, hygiene, genetics, virology, bacteriology, toxicology, pathology, environmental analysis and medical diagnosis.

28. Use of combinations of ferri- or ferromagnetic substances with structure-specific substances or of combinations of ferri- or ferromagnetic substances with analytes to be detected in methods according to Claims 1-17 and 19 to 21.

29. Use of compounds in methods according to Claims 1-17 and 19-21, characterized in that they consist of combinations of biodegradable ferri- or ferromagnetic substances with structure-specific substances, where the brownian relaxation times of the combinations of ferri- or ferromagnetic substances with structure-specific substances are shorter than the Néel-type relaxation times in body fluids at body temperature.

30. Use according to Claim 29, characterized in that the ferri- or ferromagnetic substances are iron oxides which are biodegradable.

## Revendications

1. Procédé de détection qualitative et/ou quantitative d'analytes en phases liquide et/ou solide, caractérisé en ce que des particules colloïdales ferromagnétiques ou ferrimagnétiques sont utilisées dans des immuno-dosages ou autres dosages par liaison comme marquage magnétique à détecter, et en ce que la relaxation de leur aimantation est déterminée comme grandeur mesurée.

2. Procédé de détection quantitative d'analytes en phases liquide et solide au moyen de mesures de relaxation magnétiques, caractérisé en ce que des particules colloïdales ferromagnétiques ou ferrimagnétiques sont utilisées dans des immuno-dosages ou autres dosages par liaison comme marquage magnétique à détecter, dont la relaxation brownienne dans les conditions de mesure se déroule plus rapidement que la relaxation de Néel.

3. Procédé de détection quantitative d'analytes en phases liquide et solide au moyen d'une mesure de la susceptibilité complexe en tant que fonction de la fréquence, caractérisé en ce que des particules colloïdales ferromagnétiques ou ferrimagnétiques sont utilisées dans des immuno-dosages ou autres dosages par liaison comme marquage magnétique à détecter, dont la relaxation brownienne dans les conditions de mesure se déroule plus rapidement que la réaction de Néel.

4. Procédé de détection quantitative d'analytes en phases liquide et solide, caractérisé en ce que des substances à structure spécifique liant les analytes sont d'abord
(i) marquées avec des particules colloïdales ferrimagnétiques ou ferromagnétiques, puis
(ii) ces substances à structure spécifique marquées sont utilisées dans un échantillon liquide ou immobilisé à mesurer, l'échantillon à mesurer est remagnétisé au moyen d'un champ magnétique appliqué de l'extérieur et la relaxation de l'aimantation des marqueurs magnétiques est mesurée après la coupure du champ extérieur au moyen de capteurs de champ magnétique.

5. Procédé de détection quantitative d'analytes en phases liquide et solide, caractérisé en ce que des analytes sont d'abord
(i) marqués avec des particules colloïdales ferrimagnétiques ou ferromagnétiques, puis
(ii) ces analytes magnétiquement marqués sont utilisés dans un échantillon liquide ou immobilisé à mesurer, auquel on ajoute des substances qui lient spécifiquement les analytes, et l'échantillon à mesurer est remagnétisé au moyen d'un champ magnétique appliqué de l'extérieur et la relaxation de l'aimantation des marqueurs magnétiques est mesurée après la coupure du champ extérieur au moyen de capteurs de champ magnétique.

6. Procédé de détection quantitative d'analytes liés en phase solide, caractérisé en ce que les substances à structure spécifique liant les analytes sont d'abord
(i) marquées avec des particules colloïdales ferrimagnétiques ou ferromagnétiques se relaxant dans l'intervalle de temps de la mesure, les particules colloïdales ferrimagnétiques ou ferromagnétiques étant choisies de sorte que la relaxation brownienne présente dans les conditions de mesure un temps de relaxation inférieur à la relaxation de Néel, puis
(ii) ces substances magnétiquement marquées sont utilisées dans un échantillon immobilisé à mesurer, et l'échantillon à mesurer est remagnétisé au moyen d'un champ magnétique appliqué de l'extérieur, et la relaxation de l'aimantation des marqueurs magnétiques est mesurée après la coupure du champ extérieur au moyen de capteurs de champ magnétique, le comportement de relaxation différent des marqueurs magnétiques liés à l'analyte par rapport à ceux non liés étant utilisé pour l'analyse.

7. Procédé suivant la revendication 6, caractérisé en ce que des analytes à détecter sont marqués avec des particules colloïdales ferrimagnétiques ou ferromagnétiques se relaxant dans l'intervalle de temps de la mesure, dont la relaxation brownienne présente dans les conditions de mesure un temps de relaxation inférieur à la relaxation de Néel et en ce qu'on ajoute en outre aux échantillons à mesurer les substances liant spécifiquement les analytes, ou selon le cas les substances liant spécifiquement les analytes sont présentes dans l'échantillon à l'état immobilisé.

8. Procédé de détection quantitative d'analytes présents en phase liquide, caractérisé en ce que les substances à structure spécifique liant les analytes sont d'abord
(i) marquées avec des particules colloïdales ferrimagnétiques ou ferromagnétiques, dont la relaxation brownienne présente dans les conditions de mesure un temps de relaxation inférieur à la relaxation de Néel, puis
(ii) ces substances magnétiquement marquées sont utilisées dans un échantillon à mesurer, et l'échantillon à mesurer est remagnétisé au moyen d'un champ magnétique appliqué de l'extérieur et la relaxation de l'aimantation des marqueurs magnétiques est mesurée après la coupure du champ extérieur au moyen de capteurs de champ magnétique, le comportement de relaxation différent des marqueurs magnétiques liés à l'analyte par rapport à ceux non liés étant utilisé pour l'analyse.

9. Procédé suivant la revendication 8, caractérisé en ce que des analytes à détecter sont marqués avec des particules colloïdales ferrimagnétiques ou ferromagnétiques, dont la relaxation brownienne présente dans les conditions de mesure un temps de relaxation inférieur à la relaxation de Néel, et en ce qu'on ajoute en outre aux échantillons à mesurer les substances liant spécifiquement les analytes.

10. Procédé suivant les revendications 1 et 4 à 9, caractérisé en ce qu'on utilise pour la détection la mesure de la susceptibilité complexe en tant que fonction de la fréquence.

11. Procédé suivant les revendications 1 à 10, caractérisé en ce que les substances à structure spécifique sont des anticorps, des fragments d'anticorps, de la biotine ou des substances liant spécifiquement la biotine, comme l'avidine ou la streptavidine, des agonistes se liant spécifiquement sur des récepteurs ou leurs antagonistes, des peptides et des protéines spécifiques, des récepteurs, des enzymes, des substrats enzymatiques, des nucléotides, des acides ribonucléiques, des acides désoxyribonucléiques, des hydrates de carbone ou des lipoprotéines.

12. Procédé suivant la revendication 11, caractérisé en ce que les agonistes se liant sur les récepteurs sont des cytokines, des lymphokines ou des endothélines.

13. Procédé suivant les revendications 11 et 12, caractérisé en ce que les substances à structure spécifique ont une constante de liaison dans l'intervalle de 10⁵ à 10¹⁵ (mole/l)⁻¹.

14. Procédé suivant les revendications 11 et 12, caractérisé en ce que les substances à structure spécifique ont une constante de liaison dans l'intervalle de 10⁷ à 10¹⁵ (mole/l)⁻¹.

15. Procédé suivant les revendications 1 à 14, caractérisé en ce qu'on utilise comme capteurs de champ magnétique des Superconducting Quantum Interference Devices (SQUID), des bobines d'induction, des magnétomètres à noyau saturable, des capteurs à résistance magnétique géante ou des transducteurs magnétorésistifs.

16. Procédé suivant les revendications 1 à 15, caractérisé en ce qu'on réalise une détermination de deux ou de plusieurs analytes différents dans un échantillon.

17. Procédé suivant la revendication 16, caractérisé en ce qu'on utilise deux ou plusieurs substances ferromagnétiques ou ferrimagnétiques avec des temps de relaxation brownien ou de Néel discriminables.

18. Utilisation de liaisons de détection quantitative d'analytes en phases liquide et solide au moyen de mesures de relaxation magnétiques ou au moyen de mesures de la susceptibilité complexe en tant que fonction de la fréquence, caractérisée en ce qu'elles sont constituées de combinaisons de particules colloïdales ferromagnétiques ou ferrimagnétiques avec des substances à structure spécifique ou avec des substances à détecter, dont la relaxation brownienne se déroule dans les conditions de mesure plus rapidement que la relaxation de Néel.

19. Procédé suivant les revendications 1 à 17, caractérisé en ce que les substances ferromagnétiques et ferrimagnétiques ont une taille particulaire comprise dans l'intervalle entre 1 et 400 nm.

20. Procédé suivant les revendications 1 à 17, caractérisé en ce que les substances ferromagnétiques et ferrimagnétiques ont une taille particulaire comprise dans l'intervalle entre 1 et 100 nm.

21. Procédé suivant les revendications 1 à 17, caractérisé en ce que les substances ferromagnétiques et ferrimagnétiques sont stabilisées avec une enveloppe faite d'hydrates de carbone oligomères ou polymères, de protéines, de peptides, de nucléotides et/ou de lipides.

22. Utilisation suivant la revendication 18, caractérisée en ce que les substances ferromagnétiques et ferrimagnétiques ont une taille particulaire comprise dans l'intervalle entre 1 et 400 nm.

23. Utilisation suivant la revendication 18, caractérisée en ce que les substances ferromagnétiques et ferrimagnétiques ont une taille particulaire comprise dans l'intervalle entre 1 et 100 nm.

24. Utilisation suivant les revendications 18, 22 et 23, caractérisée en ce que les substances ferromagnétiques et ferrimagnétiques sont stabilisées avec une enveloppe faite d'hydrates de carbone oligomères ou polymères, de protéines, de peptides, de nucléotides, de tensioactifs, de polymères et/ou de lipides.

25. Utilisation suivant les revendications 18, 22 et 23, caractérisée en ce que les substances à structure spécifique sont des anticorps, des fragments d'anticorps, de la biotine ou des substances liant la biotine comme l'avidine ou la streptavidine, des agonistes se liant spécifiquement sur des récepteurs ou leurs antagonistes, des peptides et protéines spécifiques, des récepteurs, des enzymes, des substrats enzymiques, des nucléotides, des acides ribonucléiques, des acides désoxyribonucléiques, des hydrates de carbone ou des lipoprotéines.

26. Utilisation des procédés suivant les revendications 1 à 17 et 19 à 21 dans la fertilité, l'histocompatibilité, l'allergologie, l'infectiologie, l'hygiène, la génétique, la virologie, la bactériologie, la toxicologie, la pathologie, l'écologie et le diagnostic médical.

27. Utilisation des liaisons suivant les revendications 18, 22 et 23 dans la fertilité, l'histocompatibilité, l'allergologie, l'infectiologie, l'hygiène, la génétique, la virologie, la bactériologie, la toxicologie, la pathologie, l'écologie et le diagnostic médical.

28. Utilisation de combinaisons faites de substances ferrimagnétiques ou ferromagnétiques avec des substances à structure spécifique ou de combinaisons faites de substances ferrimagnétiques ou ferromagnétiques avec des analytes à détecter dans des procédés suivant les revendications 1 à 17 et 19 à 21.

29. Utilisation de liaisons pour l'application dans des procédés suivant les revendications 1 à 17 et 19 à 21, caractérisée en ce qu'elles sont constituées de combinaisons de substances ferrimagnétiques ou ferromagnétiques biologiquement décomposables avec des substances à structure spécifique, les temps de relaxation browniens des combinaisons faites de substances ferrimagnétiques ou ferromagnétiques avec des substances à structure spécifique à température du corps dans des liquides du corps étant inférieurs aux temps de relaxation de Néel.

30. Utilisation suivant la revendication 29, caractérisée en ce que les substances ferrimagnétiques ou ferromagnétiques sont des oxydes de fer qui sont biologiquement décomposables.
